Europäisches Patentamt

European Patent Office

Office européen des brevets

(19)

(11) Publication number: **0 026 544**
**B1**

# EUROPEAN PATENT SPECIFICATION

(12)

(45) Date of publication of patent specification: **15.02.84**

(21) Application number: **80200908.4**

(22) Date of filing: **26.09.80**

(51) Int. Cl.³: **C 07 F 3/00, C 07 C 31/30, C 07 C 29/68 //C08G65/28, C07C41/03, B01J31/02**

(54) Process for preparing basic salts of barium.

(30) Priority: **27.09.79 US 79539**

(43) Date of publication of application:
**08.04.81 Bulletin 81/14**

(45) Publication of the grant of the patent:
**15.02.84 Bulletin 84/7**

(84) Designated Contracting States:
**BE DE FR GB IT NL SE**

(56) References cited:
EP - A - 0 001 861
EP - A - 0 026 546
DE - B - 1 166 164
FR - A - 879 689
FR - A - 1 176 300
FR - A - 1 225 135
FR - A - 1 366 307
FR - A - 1 555 094
GB - A - 767 601
GB - A - 1 501 327
US - A - 3 009 887
US - A - 3 100 750
US - A - 3 803 250
US - A - 4 020 115
US - A - 4 126 627
US - A - 4 223 164

(73) Proprietor: **UNION CARBIDE CORPORATION**
**Old Ridgebury Road**
**Danbury Connecticut 06817 (US)**

(72) Inventor: **McCain Jr., James Herndon**
**1987 Parkwood Road**
**Charleston West Virginia 25314 (US)**
Inventor: **Theiling Jr., Louis Foster**
**1408 Woodmere Park**
**Charleston West Virginia 25314 (US)**

(74) Representative: **Urbanus, Henricus Maria, Ir. et al,**
**c/o Vereenigde Octrooibureaux Nieuwe Parklaan 107**
**NL-2587 BP 's-Gravenhage (NL)**

(56) References cited:
CHEMICAL ABSTRACTS, vol. 74, no. 24, 14th June 1971, page 9, no. 126177j Columbus, Ohio, U.S.A. K.S. KAZANSKII et al.: "Polymerization of ethylene oxide in the presence of alkaline earth alcoholates" HOUBEN WEYL, Methoden der Organischen Chemie, Band VI/2, Sauerstoff-Verbindungen 1, Teil 2, 1963, pages 13-16 Stuttgart, DE Encyclopedia of Chemical Technology, Kirk-Othmen, 3rd Edition, Volume 2, pages 8-9

Courier Press, Leamington Spa, England.

Process for preparing basic salts of barium

## BACKGROUND OF THE INVENTION

This invention relates to the preparation of compositions which are catalytically active for the preparation of condensation reaction products of an epoxide and organic compounds having an active hydrogen with a restricted molecular weight distribution and reduced by-products.

A variety of products such as surfactants, functional fluids, glycol ethers, polyols, and the like are commercially prepared by the condensation reaction of epoxides with organic compounds having an active hydrogen, generally in the presence of an alkaline or acidic catalyst. The types of products prepared and properties thereof depend on the active hydrogen compound, the epoxide, and the number of moles of epoxide employed as well as the catalyst, a mixture of condensation products species being obtained containing different molecular proportions of epoxide. Thus, the reaction products generally obtained have a wide range of molecular weights and of molecular distribution of the epoxide units.

It is generally desirable to restrict the molecular distribution of the mixture to adjacent analogues of the desired product, insofar as possible, but this is quite difficult to control Acidic catalysts tend to give a narrower molecular distribution than alkaline catalysts, but also contribute to the formation of undesired by-products. Thus, alkaline catalysts are generally used as the more efficient type of catalyst but the molecular distribution in the resulting products are more diffuse. For example the use of sodium salts to catalyse the polymerisation of epoxides was disclosed in the French patents 879.689 and 1.366.307.

Heretofore, several methods have been suggested for providing reaction products of an active hydrogen compound and epoxides having a narrower range of molecular weights and molecular distribution of the epoxide units, or which reduce or eliminate the production of undesirable poly(ethylene) glycol and cyclic and straight chain ether by-products. For example, in U.S. Patent 4,112,231 to Weibull et al. it is disclosed that the use of certain neutral inorganic fluoborate and perchlorate salts will catalyze the reaction of epoxides with active hydrogen compounds to give products having a narrower molecular distribution and a larger proportion of desired species; in U.S. Patent No. 3,682,849 to Smith et al improved ethoxylated derivatives of $C_{11}$—$C_{18}$ alcohols are prepared by removing unreacted alcohol and lower ethoxylates from the conventionally produced ethoxylate mixture using vapor phase separation techniques; in U.S. Patent 2,870,220 to Carter, a two-stage process is disclosed for preparing monoalkyl ethers of ethylene glycol and polyethylene glycols of more restricted molecular weight range wherein an alkanol and ethylene oxide are reacted in the presence of an acidic catalyst during the first stage and then in the second-stage, after removal of acid catalyst and unreacted alkanol, reacting the mixture with ethylene oxide in the presence of an alkali metal alcoholate of the initial alkanol; and in U.K. Patent 1,501,327 to Laemmle et al is disclosed a method of preparing mono- and poly-glycol ethers substantially free of undesired alkylene glycol by-products which involves heating a reaction mixture containing an alkylene oxide and an alcohol in the presence of a catalyst containing alkali or alkaline earth cations wherein some or all of the catalyst is an anhydrous high boiling liquid residue prepared by concentrating the liquid residue left from the same or different etherification process after removal of the glycol ether product from the reaction mixture.

There has also been suggested basic compounds of alkali and alkaline earth metals that have been used, for example, in the polymerization of alkylene oxides (see U.S. Patent 3,100,750 to Bailey, Jr. et al and French Patent 1.176.300) and the preparation of nonionic surfactants having special properties (see U.S. Patent 4,134,854 to Milligan). To the best of our knowledge, however, none of the processes or special catalysts disclosed in the art are completely satisfactory in that they require multi-stage procedures or special acid-resistant equipment, give undesirable by-products or simply do not provide sufficient control over the molecular weight distribution. It would be highly desirable, therefore, to develop a process for the preparation of compositions which are catalytically active in the reaction of an epoxide with an organic compound having an active hydrogen wherein such reaction could be more readily carried out to prepared products that have a narrow molecular weight distribution of anologue species and contain only small amounts, at most, of undesirable by-products.

## SUMMARY OF THE INVENTION

Various soluble basic salts of alkaline earth metals have been described in EP—A—0 026 547 (Application No. 80200911.8 as being effective catalysts for the preparation of condensation reaction products of an epoxide and an organic compound having an active hydrogen compound. The present invention is directed to the novel method of preparation of a class of catalysts which are extremely effective for the preparation of oxy-alkylation reaction products having a narrower molecular weight distribution and reduced amount of undesirable by-products.

In accordance with the present invention there is provided a process for the preparation of soluble basic salts of barium that are catalytically active in the oxy-alkylation reaction of alcohols, polyols, and phenols, which comprises reacting barium oxide with an alkanol having 1 to 4 carbon atoms to form a basic barium compound, soluble in the alkanol; mixing a polyol, or an aliphatic, cycloaliphatic or phenyl-substituted aliphatic monohydric alcohol having at least 4 carbon atoms which is different from the $C_{1-4}$ alkanol with the $C_{1-4}$ alkanol-barium oxide reaction product and removing the $C_{1-4}$ alkanol from the reaction product.

It has been discovered that the soluble basic salts of barium herein described not only catalyze the reaction of an alcohol, polyol, or a phenol and an epoxide but also favor a narrower molecular distribution, i.e., a more limited range of molecular species and a larger proportion of the desired species in the reaction product. Moreover, the oxyalkylation reaction can be carried out in a single stage without the need for special acid-resistant equipment and the products produced thereby have been found, in general, to contain only small amounts of undesired poly(alkylene) glycol and ether by-products.

DESCRIPTION OF THE INVENTION

In the process of the invention barium oxide is reacted with a lower alkanol having 1 to 4 carbon atoms to form the basic barium species soluble in the lower $C_{1-4}$ alkanol, mixing a polyol or a higher aliphatic, cycloaliphatic or phenyl-substituted aliphatic monohydric alcohol having at least four carbon atoms which is different from the lower $C_{1-4}$ alkanol with the lower $C_{1-4}$ alcohol barium oxide reaction product and removing the lower $C_{1-4}$ alkanol from the reaction mixture.

The lower alkanols which can be used are primary, secondary or tertiary alkanols having 1 to 4 carbon atoms which are branched or straight chain. Exemplary of preferred alkanols are methanol, ethanol, n-propanol, iso-propanol, n-butanol, and iso-butanol.

Suitable higher monohydric alcohols having at least 4 carbon atoms that may be used in accordance with the invention can be primary and secondary aliphatic alcohols which are straight or branched chain having at least 4, and preferably 8 to thirty carbon atoms. Exemplary of such primary straight chain alcohols are n-octanol, nonanol, decanol, undecanol, dodecanol, tridecanol, tetradecanol, pentadecanol, hexadecanol, and octadecanol; and of such branched chain or secondary alcohols are 2-ethylhexanol, isooctanol, sec-octanol, and isodecanol. Particularly suitable are linear and branched primary alcohols and alcohol mixtures such as produced by the "Oxo" reaction of normal $C_3$—$C_{20}$ olefins.

Also suitable are cycloaliphatic monohydric alcohols, including, for example, cycloheptanol and cyclooctanol, as well as phenyl-substituted monohydric alcohols such as benzyl alcohol, phenylethyl alcohol, and phenylpropyl alcohol.

Polyols suitable for use in accordance with the present invention have from two to thirty carbon atoms and from two to six hydroxyl groups including for example, glycerine, glycols such as ethylene glycol, propylene glycol, butylene glycol, pentylene glycol, hexylene glycol, heptylene glycol, neopentylene glycol, decylene glycol, diethylene glycol, triethylene glycol, dipropylene glycol, tripropylene glycol, pentaerythritol, galactitol, sorbitol, mannitol, erythritol, trimethylolethane and trimethylolpropane.

The process of the invention involves, in general, two steps. In the first step, barium oxide is reacted with a lower alkanol having 1 to 4 carbon atoms for the time necessary to dissolve barium oxide therein. The temperature and pressure at which the reaction between the metal and the lower $C_{1-4}$ alkanol is conducted is not critical and can vary widely. In general, the higher the temperature the faster the rate of reaction. It is usually convenient and preferred to carry out the reaction in a temperature-pressure region where the alkanol is a liquid.

The amount of barium oxide to be reacted with the lower $C_{1-4}$ alkanol is not narrowly critical and can vary widely. However, since the lower $C_{1-4}$ alkanol is to be removed during a further step of the catalyst preparation, it is preferred to use a larger amount of metal consistent with having a mixture with reasonable handling characteristics. In general, the concentration of barium in the lower $C_{1-4}$ alkanol may vary from 0.01 to 20 percent by weight, and preferably from 0.1 to 10 percent by weight of alkanol.

In the next step of the process, the basic barium species in the lower $C_{1-4}$ alkanol is mixed with a polyol or higher aliphatic, cycloaliphatic or phenyl-substituted aliphatic monohydric alcohol having at least 4 carbon atoms and the lower $C_{1-4}$ alkanol introduced with the lower $C_{1-4}$ alkanol-barium oxide reaction product is removed from the mixture. Removal of the lower $C_{1-4}$ alkanol may be conducted by any separation means that retains the catalytic activity of the soluble basic barium salt. Preferably, distillation techniques are used, but other method such as column chromatography and fractional freezing may also be employed. The lower $C_{1-4}$ alkanol is removed from the mixture at a temperature and pressure whereby, for example, the lower $C_{1-4}$ alkanol is vaporized and the higher alcohol is not, the temperature being dependent on the pressure and types of higher alcohol and lower $C_{1-4}$ alkanols that are used in the reactions.

While a variety of higher aliphatic, cycloaliphatic or phenyl-substituted aliphatic monohydric alcohols or polyols may be employed in preparing the soluble basic barium salt, it is usually preferred to employ the alcohol or mixtures thereof or polyol that is intended to be employed in the reaction with an epoxide to be catalyzed. The amount of higher aliphatic, cycloaliphatic or phenyl-substituted aliphatic monohydric alcohol or polyol mixed with the lower $C_{1-4}$ alkanol-barium oxide reaction mixture should be sufficient to convert all of the lower $C_{1-4}$ alkanol basic salt to the soluble higher alcohol basic salt thereof and preferably at least 10 percent greater than that stoichiometrically required, calculated on the assumption that the soluble basic barium salt is the barium alkoxide of the lower $C_{1-4}$ alkanol.

Alternatively, the higher aliphatic, cycloaliphatic or phenyl-substituted aliphatic monohydric alcohol or polyol mixed with the lower $C_{1-4}$ alkanol-barium oxide reaction product is the monohydric alcohol or polyol to be oxyalkylated which is added in the amount sufficient to give the desired level of catalyst in the monohydric alcohol or polyol, after removal of the lower alcohol, for the oxyalkylation reaction. The desired concentration of catalyst in the oxyalkylation reaction mixture is not critically narrow, and can, in general, vary from 0.001 percent to 10 percent by weight of barium based on the reactive hydrogen compound.

The solution of barium salt in higher aliphatic, cycloaliphatic or phenyl-substituted aliphatic monohydric alcohol or polyol so prepared is a basic alkaline salt of barium which is soluble in the reaction products as well as in the reactants employed during the reaction of an epoxide and a reactive hydrogen compound such as a monohydric alcohol, polyol or phenol.

The soluble basic salt of barium prepared in accordance with the practice of the invention catalyzes the reaction of an organic compound having an active compound such as monohydric alcohols, polyols, and phenols with epoxides. The reaction may be conducted in a conventional manner, that is, the active hydrogen compound and the catalyst are placed in a reactor, the epoxide is added at the reaction temperature until the desired number of moles have been added, and the product is removed from the reactor and neutralized. The reaction may be conducted in the presence of a solvent, but usually a solvent is not employed.

The temperature at which the oxyalkylation reaction proceeds when the soluble basic salt of barium of the invention is employed is not narrowly critical and, generally, products can be made at a reasonable rate of reaction and without decomposition of the reactants or reaction products at a temperature between 50°C and 270°C with a temperature between 100°C and 200°C being generally preferred. While the pressure of the reaction is not narrowly critical, when low-boiling epoxides, such as ethylene oxide and propylene oxide are employed, a pressurized reactor is preferably used.

The novel catalysts of the invention are useful in effecting the oxyalkylation of primary and secondary monohydric aliphatic alcohols which are straight or branched chain and have from one to thirty carbon atoms. Exemplary of such primary straight chain monohydric alcohols are methanol, ethanol, butanol, pentanol, hexanol, heptanol, octanol, nonanol, decanol, undecanol, dodecanol, tridecanol, tetradecanol, pentadecanal, hexadecanol, and octadecanol; and of such branched chain or secondary alcohols are isopropyl alcohol, 2-ethylhexanol, sec-butanol, iso-butanol, 2-pentanol, 3-pentanol, iso-octanol, sec-octanol, and isodecanol. Particularly suitable are linear and branched primary alcohols and alcohol mixtures such as are produced by the "Oxo" reaction of normal $C_3$—$C_{20}$ olefins.

Also applicable are cycloaliphatic monohydric alcohols, including, for example, cyclohexanol, cyclopentanol, cycloheptanol, cyclopropanol and cyclooctanol, as well as phenyl-substituted nonohydric alcohols such as benzyl alcohol, phenylethyl alcohol, and phenylpropyl alcohol.

Applicable phenols include, for example phenol, and alkyl phenols such as p-methylphenol, p-ethylphenol, p-butylphenol, p-hepty-phenol, p-nonylphenol, dinonylphenol and p-decylphenol. The aromatic radicals may contain other conventional substituents such as halide atoms.

The polyols to which the present invention is applicable can have from two to thirty carbon atoms and from two to six hydroxyl groups including, for example, glycerine, glycols such as ethylene glycol, propylene glycol, butylene glycol, pentylene glycol, hexylene glycol, heptylene glycol, neopentylene glycol, decylene glycol, diethylene glycol, triethylene glycol, dipropylene glycol, tripropylene glycol, pentaerythritol, galactitol, sorbitol, mannitol, erythritol, trimethylolethane and trimethylolpropane.

The epoxides which are applicable in accordance with the invention can be any epoxide having from two to thirty carbon atoms. Exemplary epoxides include alkylene oxides such as ethylene oxide; propylene oxide-1,2; butylene oxide-1,2 and -2,3 pentylene oxide-1,2; hexylene oxide -1,2; octylene oxide -1,2; and decylene oxide -1,2; and mixtures of the same; epoxidized fatty alcohols derived from fatty oils such as epoxidized soybean fatty alcohols and epoxidized linseed oil fatty alcohols; cycloalkylene epoxides including, for example, cyclohexane oxide, cyclopentene oxide, cycloheptene oxide; aromatic epoxides such as styrene oxide and 2-methyl styrene oxide; and hydroxy-and halogen-substituted epoxides such as glycidol, epichlorohydrin and epibromhydrin.

The amount of catalyst used in accordance with the invention is not narrowly critical and a catalytic effect has been noted with only a small amount thereof being present. In general, the catalyst concentration can vary from 0.001 percent to 10 percent by weight barium based on the weight of active hydrogen compound. Concentrations of barium within the range from 0.05 percent to 5.0 percent by weight of active hydrogen compound are usually preferred. The reaction rate, however, is dependent on both temperature and catalyst concentration and to achieve a given rate, more catalyst is required at a low temperature than at a high temperature.

The invention will become more clear when considered together with the following examples which are set forth as being merely illustrative of the invention and which are not intended, in any manner, to be limitative thereof. Unless otherwise indicated, all parts and percentages are by weight.

Example 1

Barium oxide (13.6 grams) was dissolved in refluxing methanol to give, after filtration, a clear solution containing 0.33 moles of barium per 1000 grams of methanol. The methanol solution so produced was added to 650 grams (3.5 moles) 1-dodecanol in a 2 liter, 3-necked glass reaction flask equipped with a stirrer, thermometer, nitrogen inlet and connected to a vacuum pump. Methanol was removed from the mixture by distillation at 667 Pa (5 mm Hg) pressure and a final temperature of 80°C. The material remaining in the reaction flask was clear and homogeneous and had no more than traces of undissolved barium oxide. The concentration of barium metal in the 1-dodecanol was 0.105 moles barium per 1000 grams of 1-dodecanol as determined by titration with 0.1 N hydrochloric acid using a phenolphthaeline indicator.

599 grams of the 1-dodecanol-barium solution (3.22 moles of 1-dodecanol; 0.063 moles of barium) were transferred to a steel 9.5 liter autoclave equipped with a stirrer, an automatic temperature controller and a automatic feed controller. The autoclave was heated to 140°C, pressurized to 238 KPa (20 Psig) with nitrogen and then to 510 KPa (60 psig) with ethylene oxide. By automatic feed, 930 grams (21.1 moles) of ethylene oxide was added over a 0.70 hour period of time. After cookout at 140°C to completely react the ethylene oxide, the product (1529 grams) was cooled, drained from the reactor, and neutralized to a pH of 7 with acetic acid, filtered and analyzed. The analytical results are reported in Table I, below. The barium catalyst was completely dissolved in the reaction mixture until neutralization.

In a second test run, 12 grams of barium oxide was mixed with 550 grams (2.96 moles) of 1-dodecanol in a 2 liter, 3-necked reaction flask equipped as noted above. After heating at 150°C and 67 kPa (500 mm Hg) vacuum for one hour, the bulk of the barium oxide had not dissolved in the dedecanol.

The mixture of dodecanol and barium oxide was transferred to the 9.5 liter autoclave employed in test run #1 of this Example and heated and pressurized as described above. The mixture was reacted with 6.5 moles of ethylene oxide per mole of dodecanol in the same way as described in test run #1 of this Example. The reaction product produced was removed from the reactor, neutralized and filtered. Analytical results determined from the reaction product of this test run are reported in Table I, below.

In a third test run, 4.26 grams (0.076 moles) of potassium hydroxide were mixed with 525 grams (2.82 moles) of 1-dodecanol in a 1-liter glass reaction flask and heated at 110°C under 2 mm vacuum to remove any water. The solution obtained contained 0.144 moles of potassium per 1000 grams of 1-dodecanol.

A 500 gram (2.66 moles of 1-dodecanol) portion of the solution prepared above was charged to the autoclave reactor described above and a total of 757 grams (17.2 moles) of ethylene oxide were added over a period of 0.72 hours while heating at 140°C under 510 KPa (60 Psig). After a period of heating at 140°C to completely react the ethylene oxide, the reactor was cooled and the reaction mixture was drained, neutralized with phosphoric acid and filtered. Results of an analysis of the reaction product are reported in Table I, below.

Gel permeation chromatography, a standard technique for determining the molecular weight distribution of polymers and surfactants was used in the evaluation of the reaction products. A comparison of the widths of gel permeation peaks at their half-heights, all run at constant conditions, is a measure of the relative broadness of the molecular weight distribution of the polymers or surfactant. When the performance of the instrument is calibrated with standards of known molecular weights, it is possible to define the molecular weight range represented by the peak width at half height.

TABLE I

| | Catalyst | Concentration Weight Percent | Reaction Rate (a) | Molecular Weight | Cloud Point (b) °C | GEL PERMEATION CHROMATOGRAPHY RESULTS | |
|---|---|---|---|---|---|---|---|
| | | | | | | Peak Width At One-Half Height (cc) | Molecular Weight Range At One-Half Height |
| Run #1 | Barium oxide-methanol | 1.6 | 350 | 475 | 56 | 3.2 | 280–660 |
| Run #2 | Barium Oxide | 1.9 | 68 | 482 | — | — | — |
| Run #3 | Potassium Hydroxide | 0.47 | 330 | 480 | 52 | 4.5 | 270–930 |

(a) moles of ethylene oxide per mole of catalyst per hour.

(b) 1% solution ASTM D–2024.

The catalytic effect using soluble basic barium (barium oxide-methanol) and potassium hydroxide is apparent from the above results as is the much slower reaction rate obtained where barium oxide is not dissolved in the 1-dodecanol (run #2). The much more favorable molecular distribution of the reaction products obtained with the soluble basic barium catalyst as compared to potassium hydroxide is also apparent.

Example 2

A 100 cc rocking autoclave reactor equipped with an automatic temperature controller was used in this Example.

Barium oxide (0.9 grams) was added to 45 cc of ethanol refluxing at atmospheric pressure. After the barium oxide had completely dissolved, about one hour, 40 grams (.22 moles) of 1-dodecanol was added and the ethanol was removed by distillation under vacuum. The dodecanol was then held at 150°C and 67 kPa (500 mm Hg) vacuum for one hour. After cooling, the dodecanol solution was clear and homogeneous and had no more than traces of undissolved barium oxide.

The dodecanol solution containing barium (30.4 grams) was charged to a 100 cc rocking autoclave along with 21.3 grams (0.48 moles) of ethylene oxide and heated at 140°C for one hour. After cooling, the reaction product was discharged from the autoclave and analysis there showed that substantially all of the ethylene oxide had reacted.

**Claims**

1. A process for the preparation of soluble basic salts of barium that are catalytically active in the oxy-alkylation reaction of alcohols, polyols, and phenols which comprises reacting barium oxide with an alkanol having 1 to 4 carbon atoms to form a basic barium compound, soluble in the alkanol; mixing a polyol, or a higher aliphatic, cycloaliphatic or phenyl-substituted aliphatic monohydric alcohol having at least 4 carbon atoms which is different from the $C_{1-4}$ alkanol with the $C_{1-4}$ alkanol-barium oxide reaction product and removing the $C_{1-4}$ alkanol from the reaction product.

2. The process of claim 1 in which the amount of polyol or higher aliphatic, cycloaliphatic or phenyl-substituted aliphatic monohydric alcohol having at least 4 C-atoms added to the $C_{1-4}$ alkanol-barium oxide reaction product is an amount sufficient to convert all of the $C_{1-4}$ alkanol basic salt of barium.

3. The process of claim 1 in which the concentration of barium in the $C_{1-4}$ alkanol reaction mixture is from 0.01 to 20 percent by weight.

4. The process of claim 1 in which an aliphatic, cycloaliphatic or phenyl-substituted aliphatic monohydric alcohol is used having between 8 and 30 carbon atoms.

5. The process of claim 1 in which the polyol has from 2 to 30 carbon atoms and from 2 to 6 hydroxyl groups.

6. The process of claim 1 in which a polyol is mixed with the alkanol-barium oxide reaction product.

7. The process of claim 1 in which an aliphatic, cycloaliphatic or phenyl-substituted aliphatic monohydric alcohol having at least 4 carbon atoms is mixed with the $C_{1-4}$ alkanol-barium oxide reaction product.

8. A process for reacting reactive hydrogen compound and epoxides which comprises reacting a reactive hydrogen compound selected from aliphatic, cycloaliphatic or phenyl-substituted monohydric alcohols having at least 4 C-atoms, polyols and phenols with an epoxide in the presence of at least a catalytic amount of a soluble basic salt of barium prepared in accordance with the method of claim 1.

9. The process of claim 8 in which the reactive hydrogen compound is the same as the polyol or aliphatic, cycloaliphatic or phenyl-substituted aliphatic monohydric alcohol that was used in claim 1.

**Patentansprüche**

1. Verfahren zur Herstellung von löslichen basischen Bariumsalzen, die bei der Oxyalkylierung von Alkoholen, Polyolen und Phenolen katalytisch aktiv sind, umfassend die Umsetzung von Bariumoxid mit einem Alkanol mit 1 bis 4 Kohlenstoffatomen zu einer in dem Alkanol löslichen basischen Bariumverbindung; das Mischen eines Polyols oder eines höheren aliphatischen, cycloaliphatischen oder phenylsubstituierten aliphatischen einwertigen Alkohols mit mindestens 4 Kohlenstoffatomen, der von dem $C_{1-4}$-Alkanol verschieden ist, mit dem $C_{1-4}$-Alkanol-Bariumoxid-Reaktionsprodukt und das Entfernen des $C_{1-4}$-Alkanols aus dem Reaktionsprodukt.

2. Verfahren nach Anspruch 1, worin die Menge des Polyols oder höheren aliphatischen, cycloaliphatischen oder phenyl-substituierten aliphatischen einwertigen Alkohols mit mindestens 4 C-Atomen, die zu dem $C_{1-4}$-Alkanol-Bariumoxid-Reaktionsprodukt gegeben wird, eine ausreichende Menge ist, um das gesamte basische $C_{1-4}$-Alkanol-Bariumsalz umzuwandeln.

3. Verfahren nach Anspruch 1, worin die Bariumkonzentration des $C_{1-4}$-Alkanol-Reaktionsgemisches 0,01 bis 20 Gewichtsprozent beträgt.

4. Verfahren nach Anspruch 1, worin ein aliphatischer, cycloaliphatischer oder phenyl-substituierter aliphatischer einwertiger Alkohol mit zwischen 8 und 30 Kohlenstoffatomen verwendet wird.

5. Verfahren nach Anspruch 1, worin das Polyol 2 bis 30 Kohlenstoffatome und 2 bis 6 Hydroxylgruppen aufweist.

6. Verfahren nach Anspruch 1, worin ein Polyol mit dem Alkanol-Bariumoxid-Reaktionsprodukt vermischt wird.

7. Verfahren nach Anspruch 1, worin ein aliphatischer, cycloaliphatischer oder phenyl-substituierter aliphatischer einwertiger Alkohol mit mindestens 4 Kohlenstoffatomen mit dem $C_{1-4}$-Alkanol-Bariumoxid-Reaktionsprodukt vermischt wird.

8. Verfahren zur Umsetzung von Verbindungen mit reaktivem Wasserstoff und Epoxiden, umfassend die Umsetzung einer Verbindung mit reaktivem Wasserstoff, ausgewählt unter aliphatischen, cycloaliphatischen oder phenyl-substituierten einwertigen Alkoholen mit mindestens 4 C-Atomen, Polyolen und Phenolen mit einem Epoxid in Gegenwart mindestens einer katalytischen Menge eines löslichen basischen Bariumsalzes, hergestellt nach dem Verfahren von Anspruch 1.

9. Verfahren nach Anspruch 8, worin die Verbindung mit reaktivem Wasserstoff dem in Anspruch 1 verwendeten Polyol oder aliphatischen, cycloaliphatischen oder phenyl-substituierten aliphatischen einwertigen Alkohol entspricht.

**Revendications**

1. Procédé de préparation de sels basiques solubles de baryum qui sont catalytiquement actifs dans la réaction d'oxy-alkylation d'alcools, de polyols et de phénols, qui consiste à faire réagir de l'oxyde de baryum avec un alcanol ayant 1 à 4 atomes de carbone pour former un composé de baryum basique, soluble dans l'alcanol; à mélanger un polyol ou un alcool monohydroxylique aliphatique supérieur, cycloaliphatique ou aliphatique à substituant phényle, ayant au moins 4 atomes de carbone, qui est différent de l'alcanol en $C_1$ à $C_4$, avec le produit de réaction entre l'alcanol en $C_1$ à $C_4$ et l'oxyde de baryum et à éliminer l'alcanol en $C_1$ à $C_4$ du produit de réaction.

2. Procédé suivant la revendication 1, dans lequel la quantité de polyol ou d'alcool monohydroxylique aliphatique supérieur, cycloaliphatique ou aliphatique à substituant phényle ayant au moins 4 atomes de carbone ajouté au produit de réaction entre l'oxyde de baryum et l'alcanol en $C_1$ à $C_4$ est une quantité suffisante pour convertir la totalité du sel basique d'alcanol en $C_1$ à $C_4$ du baryum.

3. Procédé suivant la revendication 1, dans lequel la concentration du baryum dans le mélange réactionnel de l'alcanol en $C_1$ à $C_4$ va de 0,01 à 20% en poids.

4. Procédé suivant la revendication 1, dans lequel l'alcool monohydroxylique aliphatique, cycloaliphatique ou aliphatique à substituant phényle que l'on utilise a entre 8 et 30 atomes de carbone.

5. Procédé suivant la revendication 1, dans lequel le polyol a 2 à 30 atomes de carbone et 2 à 6 groupes hydroxyle.

6. Procédé suivant la revendication 1, dans lequel un polyol est mélangé avec le produit de réaction entre l'alcanol et l'oxyde de baryum.

7. Procédé suivant la revendication 1, dans lequel un alcool monohydroxylique aliphatique, cycloaliphatique ou aliphatique à substituant phényle ayant au moins 4 atomes de carbone est mélangé avec le produit de réaction entre l'alcanol en $C_1$ à $C_4$ et l'oxyde de baryum.

8. Procédé pour la réaction d'un composé à hydrogène réactif et d'époxydes, qui consiste à faire réagir un composé à hydrogène réactif choisi entre des alcools monohydroxyliques aliphatiques, cycloaliphatiques ou à substituant phényle ayant au moins 4 atomes de carbone, des polyols et des phénols avec un époxyde en présence d'une quantité au moins catalytique d'un sel basique soluble de baryum préparé conformément au procédé de la revendication 1.

9. Procédé suivant la revendication 8, dans lequel le composé à hydrogène réactif est le même que le polyol ou l'alcool monohydroxylique aliphatique, cycloaliphatique ou aliphatique à substituant phényle qui a été utilisé dans la revendication 1.